# EUROPÄISCHE PATENTSCHRIFT

(11) **EP 1 724 336 B1**
(45) Veröffentlichungstag und Bekanntmachung des Hinweises auf die Patenterteilung: **03.12.2008**
(21) Anmeldenummer: 06007487.9
(22) Anmeldetag: 10.04.2006
(51) Int. Cl.: C12N 1/04

(54) **Verfahren zur Verbesserung der Trocknungs-und Produkteigenschaften von Mikroorganismen**
Method for improving the drying and product quality of microorganisms
Procédé pour améliorer la qualité de séchage et de produit de microorganismes

(30) Priorität: 19.05.2005 AT 8542005
(43) Veröffentlichungstag der Anmeldung: 22.11.2006
(73) Patentinhaber: Fricko, Paul, Dr., 1120 Wien (AT)
(72) Erfinder: Fricko, Paul, Dr., 1120 Wien (AT)

(56) Entgegenhaltungen:
- EP-A- 0 167 643
- EP-A- 0 616 030
- WO-A-03/090543

## Beschreibung

Die Technologie zur Herstellung von getrockneten Mikroorganismen unter weitgehendem Erhalt der Vitalität ist bekannt und wird für Backhefe im industriellen Maßstab genutzt. Eine typische Beschreibung des Prozesses findet sich bei (1); ebenso werden auch spezielle Hefestämme ("Weinhefe") zur Beimpfung von Fermentationen zur Weinproduktion industriell getrocknet (2). Die verwendete Technologie ist derjenigen für Trockenbackhefe ähnlich. Essentielles Ziel dieser Trocknung ist es, die Haltbarkeit der Mikroorganismen auf diese Weise zu verlängern, was natürlich mit dem Ziel des Erhaltes der gewünschten Aktivität verbunden ist.

Allgemein kann dies so beschrieben werden, dass nach ausreichender Vermehrung der Biomasse (Mikroorganismen) in einem oder mehreren aufeinander folgenden industriellen Fermentationsschritt(en) die so gewonnene Biomasse durch Separation mit ggf. integrierten Reinigungs- (Wasch-) stufen zu einer konzentrierten wässrigen Suspension mit etwa 18 - 22 % Trockensubstanzgehalt aufgearbeitet wird. Diese Suspension wird durch Filtration (Vakuumfilter oder Druckfilter) weiter entwässert, die resultierende pastöse oder krümelige Masse durch Extrusion zu zylindrischen Strängen mit Durchmesser von etwa 0,3 - 1 mm ausgeformt und diese in einem Wirbelschichttrockner unter Einhaltung präziser Temperaturbedingungen getrocknet. Zur Verbesserung der Wasserabgabefähigkeit und der Extrusion, sowie zur möglichst weitgehenden Erhaltung der Vitalität des Produktes, werden vor der Filtration, spätestens jedoch vor der Trocknung Emulgatoren zugesetzt in einer Menge von vorzugsweise etwa 1 %, von denen sich insbesondere Sorbitanmonostearat (="SMS") als Standard etabliert hat. Es wurden auch Mischungen von Emulgatoren für den Zweck der Verbesserung der Produkteigenschaften von aktiver Trockenhefe patentiert.

In EP 0 167 643 B1 (3) wird ein Zusatzmittel für die Herstellung von aktiver Trockenhefe beschrieben, welches unter anderem Sorbitanmonostearat und Diacetylweinsäureester beinhaltet, allerdings aus mindestens vier Komponenten besteht.

In EP-A-0 616 030 (4) wird die Verbesserung der Produkteigenschaften von Trockenhefe durch Zugabe eines Rehydratationsstoffes offenbart, der mindestens 50 Gew.-% eines Emulgators beinhaltet, wie beispielsweise Sorbitanmonostearat oder Diacetylweinsäureester; Lecithin oder hydrolysiertes Lecithin werden nicht als Komponenten beschrieben.

WO 031090543A (5) offenbart die Herstellung einer Trockenheferezeptur, welche ein Hilfsmittel zur Herstellung, wie beispielsweise Sorbitanmonostearat, und ein Hilfsmittel zur Verarbeitung, wie beispielsweise Diacetylweinsäureester und/oder Lecithin beinhaltet; allerdings werden zu den Hilfsmitteln keine genauen Mengenangaben gemacht

Trotz Einsatz von Emulgatoren - insbes. SMS - kommt es während des Trocknungsprozeses zu einer Schädigung bzw. einer Abtötung eines Teiles der eingesetzten Biomasse, was sich in einer reduzierten Aktivität (bezogen auf eingesetzte Trockenmasse), sowie Haltbarkeit des Produktes äußert (2).

Aufgabe der vorliegenden Erfindung war daher die Verbesserung der Produkteigenschaften von getrockneten Hefen, insbesondere Wein- und Backhefen. Die Aufgabe wurde unter weitgehender Beibehaltung der Produktionstechnologie dadurch gelöst, dass geeignete Mischungen von Emulgatoren im Herstellungsprozess eingesetzt werden, die eine verbesserte Schutzwirkung für die Mikroorganismen darstellen und damit eine verbesserte Wasserabgabe, sowie verbesserte Restaktivität nach der Trocknung ergeben. Die Herstellung dieser Mischungen kann sowohl durch Zubereiten einer Mischung der Einsatzkomponenten als Mischemulgator und anschließende gemeinsame Emulsion in Wasser, wie auch durch einzelne, aufeinander folgende Emulsion der Einsatzkomponenten in Wasser zur Bereitung der Einsatzemulsion, erfolgen.

Bei diesen Mischungen handelt es sich um binäre oder ternäre Mischungen handelsüblicher Emulgatoren, die aus den Komponenten
- Sorbitanmonostearat (SMS)
- Lecithin
- hydrolysiertes Lecithin
- Diacetylweinsäureester
zusammengesetzt sind und als eine Hauptkomponente mindestens 30 Gew.-% SMS (Sorbitanmonostearat) enthalten.

In geeigneten Mischungen weisen diese Emulgatoren eine deutlich verbesserte Schutzwirkung als jede der Einzelkomponenten auf, insbesondere auch als Sorbitanmonostearat alleine, das derzeit als Schutzstoff eingesetzt wird. Die Einsatzmenge der erfindungsgemäßen Emulgatormischung hat sich in derselben Menge wie das derzeit eingesetzte Sorbitanmonostearat bewährt und liegt bei 0,2 - 2 %, vorzugsweise 0,5 - 1,5 % und besonders bevorzugt bei 1 %.

Als besonders vorteilhaft erwiesen sich ternäre Mischungen von Sorbitanmonostearat (40-80 Gew.-%), Lecithin (10-30 Gew.-%), und hydrolysiertem Lecithin (10-30 Gew.-%), wie auch ternäre Mischungen von Sorbitanmonostearat (40-80 Gew.-%), Lecithin (10-30 Gew.-%), und Diacetylweinsäureester (10-30 Gew.-%), und binäre Mischungen von Sorbitanmonostearat (33-67 Gew.-%), und hydrolysiertem Lecithin (67-33 Gew.-%).

Die Überprüfung der Schutzwirkung der Emulgatormischungen erfolgte durch identische Verarbeitung einer kommerziellen Hefecharge (aus einer industriellen Fermentation) zu Trockenhefe, wobei als einziger Unterschied die unterschiedliche Zusammensetzung des Emulgators bzw. der Emulgatormischung variiert wurde.

Generell wurde in den Laborversuchen Heferahm (kommerzielle Backhefe) mit dem zu testenden Emulgator bzw. Emulgatormischung (jeweils in 10%-iger wässriger Suspension) versetzt; die Einsatzmenge betrug jeweils 1% Emulgator bzw. Emulgatormischung bezogen auf Hefe (jeweils Trockensubstanz); der Emulgator bzw. Emulgatormischung wurde jeweils 10 min. im Heferahm durch Rühren verteilt, anschließend durch Druckfiltration ein festes Produkt hergestellt; dieses wiederum wurde entsprechend der industriellen Praxis zu Strängen mit Durchmesser von ca. 0,55 mm extrudiert und davon eine exakt gewogene Menge in einem Laborwirbelschichttrockner getrocknet. Die Steuerung der Trocknung erfolgte automatisch durch Regelungssystem, das in einer ersten Phase die Produkttemperatur kontrollierte, in einer zweiten Phase die Temperatur der Zuluft kontrollierte und schließlich automatisch nach Erreichen/Unterschreiten einer vorgegebenen Abluftfeuchte die Trocknung beendete.

Unter den eingestellten Trocknungsbedingungen wies das Produkt (im Falle von Sacch. Cer.) in der Folge einen TS-gehalt von mehr als 95-96 % auf. Die so hergestellte Trockenhefe wurde auf Ihre Triebkraft untersucht, was in einem Fermentograph der Fa. SJA, gemäß industrieller Praxis erfolgte; ebenso wurde ein Teil des Produktes vakuumverpackt und (zur Simulation einer lang dauernden Lagerung) thermostatisiert bei erhöhter Temperatur (72 h bei 55°C) gelagert; anschließend wurde erneut die Triebkraft bestimmt ("Triebhaltbarkeit"). Die Triebkraftbestimmung erfolgte in einem Teig mit sehr geringem Zuckerzusatz ( 2 g Zucker bei 280 g Mehl, = "Normalteig", Abkürzung "NT"), sowie fallweise in einem "Zuckerteig" (42g Zucker bei 280 g Mehl, = "Zuckerteig", Abkürzung "ZT")

### Beispiel 1:

Es wurde kommerzieller Backheferahm aus einer Fermentationscharge mit handelsüblichem Emulgator (SMS) versetzt und in der oben beschriebenen Weise zu Trockenhefe im Labor verarbeitet; ebenso wurden Vergleichsprodukte mit Emulgatormischungen gemäß einer als vorteilhaft erkannten Mischung getrocknet und auf ihre Triebkraft und Haltbarkeit untersucht. Die Ergebnisse zeigt die folgende Tabelle1

**Tabelle 1**

| Emulgator | SMS | SMS - Hydrol. Lecithin | SMS - Hydrol. Lecithin - Lecithin | SMS - Lecithin - Diacetylweinsäureester |
|---|---|---|---|---|
| Anteile in Mischung [Masse] | - | 0,5 - 0,5 | 0,6 - 0,2 - 0,2 | 0,6 - 0,2 - 0,2 |
| Einsatzmenge Emulgator | 1% | 1% | 1% | 1 % |
| Triebkraft NT *) | 685 + 975 = 1660 | 695 + 975 = 1670 | 695 + 975 = 1670 | 765 + 1020 = 1785 |
| Triebkraft ZT*) | 270 + 495 = 765 | 290 + 520 = 790 | 285 + 560 = 845 | 315 + 560 = 875 |
| Triebhaltbarkeit NT*) | 445 + 740 = 1185 | 535 + 815 = 1350 | 555 + 830 = 1385 | 580 + 875 = 1455 |
| Triebhaltbarkeit ZT*) | 170 + 330 = 500 | 180 + 375 = 555 | 215 + 425 = 640 | 230 + 450 = 680 |

| | | | | |
|---|---|---|---|---|
| *) Zahlenangeben bedeuten gebildetes CO2 nach 1 bzw. 2 Stunden und die resultierende Summe | | | | |

### Beispiel 2

In einem ähnlichen Versuch wurde mit handelsüblichem Backheferahm der Einfluss der Zusammensetzung der Emulgatormischung hinsichtlich der Konzentration der einzelnen Komponenten untersucht; die Versuchsdurchführung erfolgte wie im Beispiel 1 beschrieben, die Resultate zeigt die folgende Tabelle 2

**Tabelle 2**

| Die Einsatzmenge Emulgator bzw. Emulgatormischung betrug in allen Fällen 1% | | | | |
|---|---|---|---|---|
| Emulgator | SMS | SMS - Lecithin - Diacetylweinsäuree. | SMS - Lecithin - Diacetylweinsäuree. | SMS - Lecithin - Diacetytweinsäuree. |
| Anteile in Mischung [Masse] | - | 0,6 - 0,2 - 0,2 | 0,8 - 0,1 - 0,1 | 0,5 - 0,25 - 0,25 |
| Triebkraft NT *) | 685 + 955 = 1640 | 765 + 985 = 1750 | 750 + 975 = 1725 | 730 + 970 = 1700 |
| Triebkraft ZT*) | 245 + 480 = 725 | 275 + 530 = 805 | 255 + 485 = 740 | 280 + 535 = 875 |
| Triebhaltbarkeit NT*) | 520 + 825 = 1345 | 590 + 870 = 1460 | 590 + 865 = 1455 | 565 + 855 = 1420 |
| Triebhaltbarkeit ZT*) | 210 + 410 = 620 | 230 + 445 = 675 | 225 + 435 = 660 | 215 + 425 = 640 |

| | | | | |
|---|---|---|---|---|
| *) Zahlenangeben bedeuten gebildetes CO₂ nach 1 bzw. 2 Stunden und die resultierende Summe | | | | |

### Beispiel 3

In einem industriellen Versuch wurden die als vorteilhaft erkannten Emulgatormischungen im Vergleich mit dem üblichen SMS als Schutzstoffe zur Trocknung von Weinhefe eingesetzt. Die angewendete Technologie war für alle Schutzstoffe identisch und derjenigen der Laborversuche mit Backhefe sehr ähnlich; an Stelle der Druckfiltration wurde Vakuumfiltration zur Entwässerung des Heferahmes eingesetzt und die Aufgabe des Emulgators bzw. der Emulgatormischung (jeweils 1 %) erfolgte nicht in den Heferahm, sondern in die filtrierte Hefe. Die wesentlichen erzielten Daten und Qualitätsparameter zeigt die folgende Tabelle 3, wobei zur Analyse Mischmuster aus den durchgeführten Trocknungschargen verwendet wurden.

**Tabelle 3**

| Emulgator | SMS | SMS - Lecithin - Diacetylweinsäureester | SMS - Hydrol. Lecithin - Lecithin | SMS - Hydrol. Lecithin |
|---|---|---|---|---|
| Anteile in Mischung [Masse] | - | 0,6 - 0,2 - 0,2 | 0,6 - 0,2 - 0,2 | 0,5 - 0,5 |
| Anzahl Chargen | 42 | 60 | 57 | 31 |
| Durchschnittl. Trocknungsdauer, [min.] | 48 | 39 | 37 | 28 |
| Totzellenanteil, [%] | 58 | 34 | 46 | 45 |
| Triebkraft, NT*) | 215 + 435 = 650 | 250 + 490 = 740 | 295 + 550 = 845 | 225 + 425 = 650 |
| Triebhaltbarkeit NT*) | 75 + 170 = 245 | 150 + 275 = 425 | 145 + 310 = 455 | 130 + 250 = 380 |

| | | | | |
|---|---|---|---|---|
| *) Zahlenangeben bedeuten gebildetes CO₂ nach 1 bzw. 2 Stunden und die resultierende Summe | | | | |

### Literatur:

(1) - B. H.Lee , "Fundamentals of Food Biotechnology". 1996, VCH Publishers,
(2) - G. Reed, T.W. Nagodawithana, "Yeast Technology", 1991, Van Nostrand Reinhold
(3) - EP 0 167 643 B1
(4) - EP-A-0 616 030
(5) - WO 03/090543A

## Patentansprüche

1. Verfahren zur Herstellung von Trockenhefe, insbesondere Wein- und Backhefen, ***dadurch gekennzeichnet,* dass** spätestens vor der Trocknung den Mikroorganismen eine Emulgatormischung zugesetzt wird, die entweder aus einer ternären Mischung aus mindestens 30 Gew.- % Sorbitanmonostearat sowie 10 - 40 Gew.- % weiteren Komponenten besteht, wobei die weiteren Komponenten Lecithin und hydrolysiertes Lecithin oder Lecithin und Diacetylweinsäureester sind, oder aus einer binären Mischung, bestehend aus Sorbitanmonostearat und hydrolysiertem Lecithin mit mindestens 30 Gew.-%. Sorbitanmonostearat und 10-70 Gew.- %an hydrolysiertem Lecithin.

2. Verfahren nach Anspruch 1. ***dadurch gekennzeichnet*, dass** eine binäre Emulgatormischung aus Sorbitanmonostearat und Hydrolysiertem Lecithin mit einem Mengenanteil von 20-70 Gew.- % an Hydrolysiertem Lecithin verwendet wird.

3. Verfahren nach Anspruch 1. ***dadurch gekennzeichnet,* dass** der Mengenanteil an Sorbitanmonostearat in der ternären Mischung zwischen 40 - 80 Gew.- % liegt und der Anteil an Lecithin und hydrolysiertem Lecithin jeweils 10-30 Gew.- % beträgt.

4. Verfahren nach Anspruch 1. ***dadurch gekennzeichnet,* dass** der Mengenanteil an Sorbitanmonostearat in der ternären Mischung zwischen 40 - 80 Gew.- % liegt und der Anteil an Lecithin und Diacetylweinsäureester jeweils 10-30 Gew.- % beträgt.

## Claims

1. A method for the production of Dry Yeast, especially Wine and Baker's yeast, wherein an emulsifier mixture is added to the microorganisms latest before the drying step, which consists either of a ternary mixture with at least 30 wt% Sorbitane monostearate and 10 - 40 wt% others, which either are composed of Lecithine and hydrolysed Lecithine or of Lecithin and Diacetyl Tartaric ester, or of a binary mixture consisting of Sorbitane monostearate and hydrolysed Lecithine with at least 30 wt% Sorbitane monostearate and 10 - 70 wt% of hydrolysed Lecithine

2. A method for the production of Dry Yeast according to claim 1, wherein a binary emulsifier mixture consisting of Sorbitane monostearate and hydrolysed Lecithine with 20 - 70 wt% of hydrolysed Lecithine is applied

3. A method for the production of Dry Yeast according to claim 1, wherein the portion of Sorbitane monostearate in the ternary mixture consists of 40 -80 wt% and the portion of hydrolysed Lecithine and Lecithine each consists of 10-30 wt%

4. A method for the production of Dry Yeast according to claim 1, wherein the portion of Sorbitane monostearate in the ternary mixture consists of 40 -80 wt%. and the portion of Lecithine and Diacetyl Tartaric ester each consists of 10-30 wt%

## Revendications

1. Procédé pour la fabrication de levures séchés, en particulier des levures de vinification et de panification, **caractérisé par** l' addition, au plus tard avant le séchage, soit d'un mélange d'émulsifiants ternaires, qui se composent d'au moins 30 pour cent masse de monostéarate de sorbitane et de 10 à 40 pour cent masse d'autres composés, dont la lécithine et la lécithine hydrolysée ou la lécithine et l'ester d'acide tartrique diacétyle, ou d'un mélange d'émulsifiants binaires qui se composent d'au moins 30 pour cent masse de monostéarate de sorbitane et de 10 à 70 pour cent masse lécithine hydrolysée

2. Procédé pour la fabrication de levures séchés selon la spécification 1, **caractérisé par** l'utilisation d'un mélange d'émulsifiants binaires qui se composent de monostéarate de sorbitane et de 20 à 70 pour cent masse lécithine hydrolysée

3. Procédé pour la fabrication de levures séchés selon la spécification 1, **caractérisé par** l'utilisation d'un mélange d'émulsifiants ternaires ou le taux de monostéarate de sorbitane dans le melange est de 40 à 80 pour cent masse et le taux de la lécithine et de la lécithine hydrolysée est de 10 à 30 pour cent masse

4. Procédé pour la fabrication de levures séchés selon la spécification 1, **caractérisé par** l'utilisation d'un mélange d'émulsifiants ternaires ou le taux de monostéarate de sorbitane dans le melange est de 40 à 80 pour cent masse et le taux de la lécithine et de l'ester d'acide tartrique diacétyle est de 10 à 30 pour cent masse
